(19) **Europäisches Patentamt**

**European Patent Office**

**Office européen des brevets**

(11) **EP 1 067 985 B1**

(12) **EUROPEAN PATENT SPECIFICATION**

(45) Date of publication and mention
of the grant of the patent:
**16.11.2005 Bulletin 2005/46**

(51) Int Cl.⁷: **A61N 1/30**, A61B 5/05

(86) International application number:
**PCT/SE1999/000511**

(21) Application number: **99918408.8**

(22) Date of filing: **30.03.1999**

(87) International publication number:
**WO 1999/052589 (21.10.1999 Gazette 1999/42)**

(54) **AN APPARATUS FOR CONTROLLING THE GENERATION OF ELECTRIC FIELDS**

GERÄT ZUR STEUERUNG DER GENERIERUNG ELEKTRISCHER FELDER

APPAREIL PERMETTANT DE COMMANDER LA PRODUCTION DE CHAMPS ELECTRIQUES

(84) Designated Contracting States:
**AT BE CH CY DE DK ES FI FR GB GR IE IT LI LU
MC NL PT SE**
Designated Extension States:
**AL LT LV MK RO SI**

(30) Priority: **31.03.1998 SE 9801139**

(43) Date of publication of application:
**17.01.2001 Bulletin 2001/03**

(73) Proprietor: **Aditus Medical AB
227 36 Lund (SE)**

(72) Inventors:
• **PERSSON, Bertil
S-224 75 Lund (SE)**
• **BÖHMER, Bernt
S-240 36 Stehag (SE)**
• **THORVINGER, Bo
S-216 20 Malmö (SE)**

(74) Representative: **Asketorp, Göran et al
Ström & Gulliksson AB
P.O. Box 4188
203 13 Malmö (SE)**

(56) References cited:
WO-A1-93/23112          WO-A1-97/07853
WO-A1-97/07854          US-A- 5 300 068
US-A- 5 370 645         US-A1- 4 141 359
US-A1- 5 527 357

**Description**

The present invention relates to an apparatus for generating pulses of electric fields in a restricted area of a human or an animal according to the preamble to the appended independent Claim.

The therapy forms which are routinely employed in modern medical care for tumor therapy are examples of treatment types where the outcome of such treatment is unsatisfactory. For example, in tumor therapy unsuccessful attempts are often made to achieve local tumor control, which is the cause of mortality of approximately 30% of cancer patients. It is, therefore, important to develop a new and improved technique for local and regional tumor treatment.

In today's medical care, surgery, chemotherapy and radiation therapy, also known as radiation treatment, or combinations hereof are the most commonly employed methods for treating malignant tumors. Approximately every second patient suffering from infiltrating cancer is treated with radiation therapy, but only roughly half of the patients are cured. This failure is, on the one hand, the cause of the presence of wide-spread disease (distal metastasis) or relapses (the return of tumors in the treated area), and on the other hand because certain types of tumor are resistant to radiation treatment or chemotherapy.

With varying success, attempts have been made to reinforce and improve the efficiency of radiation therapy in sterilizing tumors. For example, use has been made of more sophisticated radiation therapy techniques, such as stereotactic treatment, "conformal radiotherapy", of altered fractioning or added pharmaceuticals to increase the radiation sensitivity of the tumors.

Use is also made of heat as an adjuvant ionizing radiation, which, for certain tumor forms, may increase the number of complete remissions by up to a factor of two.

Also in certain purely medically treated diseases in local organs, the outcome of treatment is occasionally insufficient. It is obvious that, in addition to the wishes which exist regarding improved techniques for treating, for example, tumors, there are not only wishes but also needs for a more efficient technique for treating certain other diseases. In, for example, the local treatment of local organs or tumors, it is a major advantage if, on each treatment occasion, it is possible to adapt the intensity of the treatment to suit the status of the tissue in the local region or in the organ being treated.

According to the present invention, use is made of a series of brief high voltage pulses for generating electric fields in the local region or in the organ which is to be treated. In the continuation of this description, use will also be made of the expression High Voltage Impulse Therapy, occasionally abbreviated to HVIT.

The treatment with electric fields realizes a perforation of the cell membranes which thereby allow the passage of substances (e.g. cytostatic or genetic material) added to the body. The treatment involves increased inflow of therapeutic substances, whereby the effects of chemotherapy are amplified. The outflow of specific substances out of, for example, tumor cells moreover often realizes a stimulation of the immune system. In total dielectric collapse, the result is often achieved that the cells are sterilized directly by the electric fields formed by the high voltage pulses. In clinical experiments, the method has proved to be effective in combination with cytostatics (Bleomycin) for, for example, treating melanoma and tumors in the neck, head, liver, pancreas and lungs.

In HVIT, the treatment result is determined by the number and duration of the high voltage pulses to which the tissue is subjected and how high electric fields the impressed pulses create in the tissue, as well as the form or frequency the pulses possess. In order to achieve an effective and dependable treatment, it must be possible to control all of these physical parameters. Biological properties which affect the treatment result are, among other things, the electric conductive capacity of the tissue, its dielectric properties, the cell sizes and the structures of the cell membranes. All of these properties vary between different tissues. In order to achieve optimum treatment effect, it is therefore necessary to measure how the electric properties of the tissue change between each high voltage pulse or between the series of pulses, i.e. to establish when the cells are sufficiently perforated.

In previously employed HVIT, it was not possible to monitor when the tissue was sufficiently perforated, i.e. when the treatment was completed, which entailed that the tissue was occasionally undertreated and occasionally overtreated. This involved a degree of uncertainty in the treatment result. A typical HVIT treatment according to prior art techniques entails that an applicator was placed over the tissue which was intended for treatment. The high voltage generator was, for example, set such that the outgoing voltage corresponded to a field in the target volume of approx. 1300 V/cm. The treatment was completed with a fixed number of pulses which it was known normally gave the desired result. The weaknesses in this procedure were, on the one hand, that the size of the electric field which the generator in reality generated in the tissue of the target volume was unknown, and, on the other hand, that it was not possible to assess when the treatment was sufficient.

Devices according to the preamble of claim 1 are disclosed in documents US-A-5 370 645 and US-A-5 300 068.

The present invention is defined in claim 1. It relates to an apparatus which includes mechanical devices for subjecting a tissue within a restricted region or an organ in a person or an animal for one or more pulses of an electric field at a field strength, configuration, duration and frequency adjustable for the relevant treatment occasion. The expression "duration" relates to both the length of the pulses and the number of pulses, the expression "frequency" relates to both how often the pulses are repeated and the frequency with which the field alternates during an ongoing pulse.

The present invention allows a technique which entails a substantial improvement to the efficiency of surgery, chemotherapy and radiation therapy. The technique is also applicable within modern molecular medicine where substances and genetic DNA sequences which are to be introduced into tissue cells are customized.

Further preferred embodiments of the present invention are disclosed in the appended subclaims.

The present invention will now be described in greater detail hereinbelow, with reference to a number of Figures, in which:

Fig. 1 is a block diagram of a fundamental apparatus for applying electric fields in a restricted region of a person or an animal;

Fig. 2 is a block diagram of a fundamental apparatus for applying electric fields and/or ionizing radiation in a restricted region of a human or an animal;

Fig. 3 is a block diagram of one embodiment of a combination of devices for forming electric fields in a restricted region of a human or an animal;

Figs. 4a-d are embodiments of electrode applicators for external treatment of tissue;

Fig. 5 shows one embodiment of an electrode applicator for intraoperative treatment of, for example, tumors and superficial tumor nodules;

Figs. 6a-d show embodiments of electrodes and electrode applicators designed for interstitial treatment of tissue;

Figs. 7a-c show embodiments of electrodes and electrode applicators designed for the treatment of, for example, tumors in bodily cavities and in organs accessible via large vessels;

Fig. 8 shows embodiments of electrodes in which these are disposed for combination treatment with antitumoral pharmaceutics;

Figs. 9a-e show examples of configurations of voltage pulses applied to the electrodes;

Fig. 10 is a simplified block diagram of one embodiment of the apparatus;

Fig. 11a shows a model of the principle structure of living tissue;

Fig. 11b is an electric skeleton diagram of the electric structure of living tissue; and

Fig. 12 is an electric model of a pulse generator connected to living tissue.

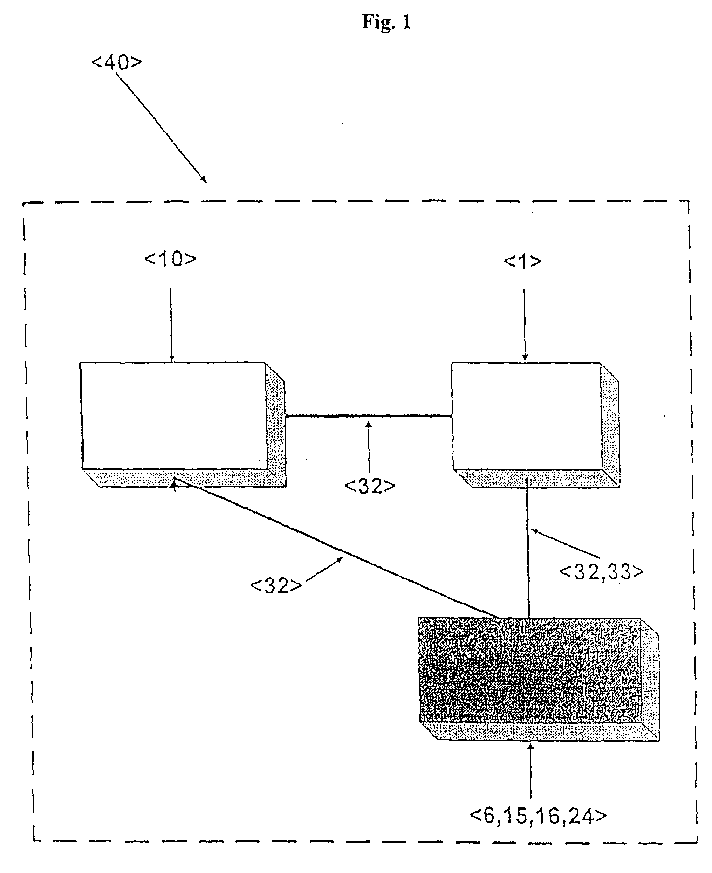

Fig. 1 shows, in block diagram, the basic design of a high voltage generator 1, electrodes 6,15,16,24 and a registration and conversion device 10, for example a computer or a microprocessor 10, these devices all being included in the apparatus according to the present invention. Hereafter, the word computer will also be employed without any restrictive intent for the registration and conversion device. Between the high voltage generator 1 and the electrodes 6,15,16,24, there are disposed one or more signal connections 32 and electric conductors 33. Between the computer 10 and the high voltage generator 1, and between the computer and the electrodes 6,15,16,24, there is provided one or more signal connections 32. While the signal connections 32 in the Figure are shown as directly connecting the computer and the electrodes, it will be obvious that the apparatus as such also includes devices described in the continued description of this application, such as switches 3, distributor 4, electrode applicator 5, etc. for controlling the voltage impressment of the electrodes, etc.

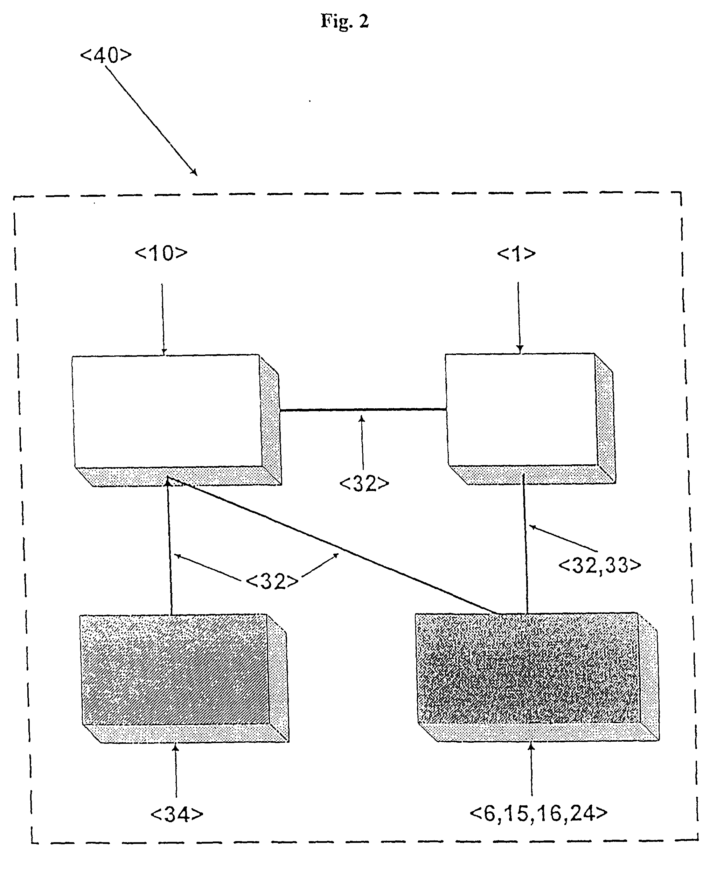

Fig. 2 shows one embodiment of the present invention in which a radiation transmitter 34 is connected via signal connections 32 to the computer. In certain embodiments, the radiation transmitter is mechanically interconnected to the high voltage generator, while in other embodiments it only has signal connection with the combination of devices illustrated in Fig. 1.

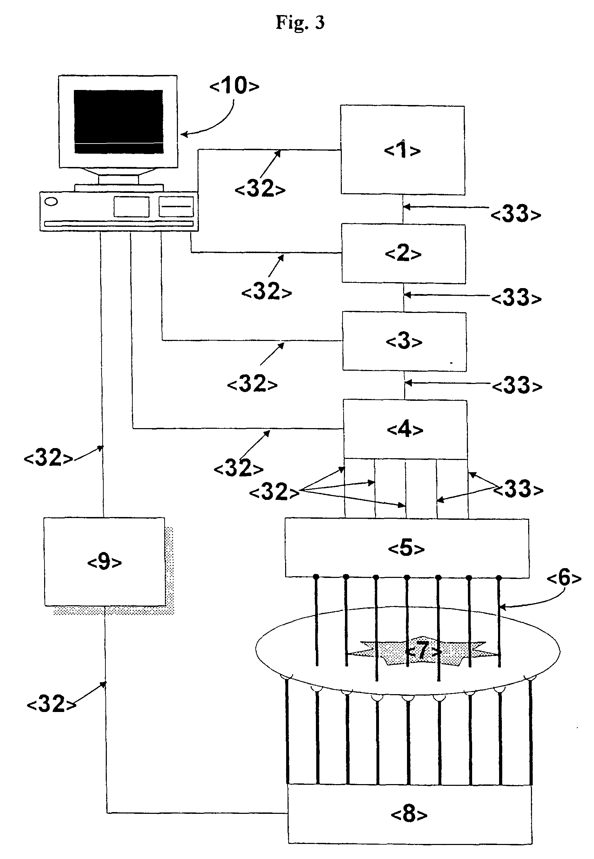

Fig. 3 schematically shows one embodiment of a combination of devices for generating electric fields according to the present invention. The Figure shows blocks for a high voltage generator 1, a capacitor battery 2, a switch 3, a distributor 4 for distributing the high voltage pulses which are generated on discharge of the capacitor battery 2 through the switch 3 to an electrode applicator 5 and electrodes 6 intended to be placed in or adjacent the tissue region 7 or organ 7 of a patient undergoing treatment. The high voltage generator 1, the capacitor battery 2, the switch 3 and the distributor 4 are connected in series with one another by means of electric conductors 33. Between the distributor 4 and the electrode applicator 5, there is provided at least one electric conductor 33 and at least one signal connection 32. Via the signal connections 32, the distributor 4 controls the voltage impression of the electrodes of the electrode applicator, via which the electric conductors 33 are interconnected to the distributor 4 and via the electric conductor 33 to the switch 3. In one alternative embodiment, each electrode 6 is electrically connected to the switch 3 by means of an electric conductor 33.

As a rule, the distributor 4 or an electrode applicator impresses voltage simultaneously on only two electrodes 6, while the other electrodes are permitted to assume that potential which is determined by the placing of the electrode in the treatment region. The term voltage impression also includes in this context the fact that one or more electrodes are earthed (have zero potential). The switch 4 and/or the electrode applicator 5 are disposed to permit, if so wished, the voltage impression pairwise of all electrodes which are placed in the treatment region. It will be obvious to a person skilled in the art, that, in certain embodiments, the devices are provided in order, on voltage impression, to allocate to several electrodes a substantially corresponding (the same) potential.

All units are, via signal connections 32 which, in certain embodiments, are wholly or partly wireless, connected to a registration and conversion device 10 with a screen 10a. Hereafter, the designations control and conversion unit 10 or computer 10 will be employed for the registration and conversion device. The computer 10 constitutes a control and monitoring device for the function of the apparatus.

The expression electrode applicator 5 relates to a retainer member for the electrodes 6, where the retainer member is designed to facilitate the correct application of the electrodes at or in the treatment region.

The computer is set as a rule for the high voltage pulses to contain the following data:

| repetition frequency | approx. 0.1-10000 per second |
|---|---|
| amplitude | approx. 50-6000V |
| pulse length | approx. 0.1-200 ms |
| number of pulses | 1-2000 per treatment. |

The pulses are applied before, during or immediately after the radiation treatment. Examples of pulse configuration employed are square pulse with a pulse length of 0.1-2 ms or exponentially fading pulse with a time constant RC approximately equal to 0.1-2 ms. In large amplitudes of the voltage, shorter pulse lengths are generally selected, and vice versa.

The high voltage generator 1 is, as a rule, disposed to emit modulated a.c. voltage of a frequency within a range of 40 Hz-2 MHz and as a rule within the range of 40 Hz-100 kHz. In those embodiments where the high voltage generator is disposed to emit a.c. voltage of high frequency, a modulator is employed instead of a capacitor battery and switch for generating short modulated high frequency pulses with a pulse length within the range of approx. 0.1-200 ms.

As will be apparent from the embodiment illustrated in Fig. 3, the apparatus generally also includes sensors 8 intended to be applied to the patient in the treatment region. The sensors are connected via a detector interface 9 to the registration and conversion device 10. On application of the treatment pulse, a signal is generated in the sensors 8 which, via the interface 9, is transferred to and registered in the computer 10. From the measured signals, the computer calculates the electric field induced by the pulse and the electromotive force in different parts of the treatment region 7. These signals entail that the computer 10 emits signals to the high voltage generator/capacitor battery (feedback) to adjust the amplitude of the generated pulses so that the predetermined field is achieved in the treatment region. This monitoring and adjustment take place continuously during the application of the pulses.

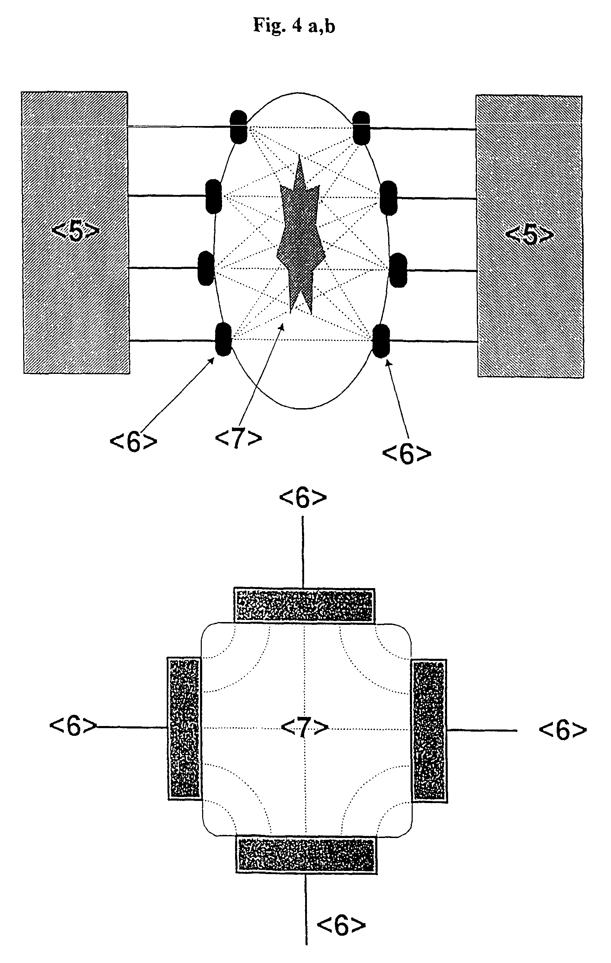

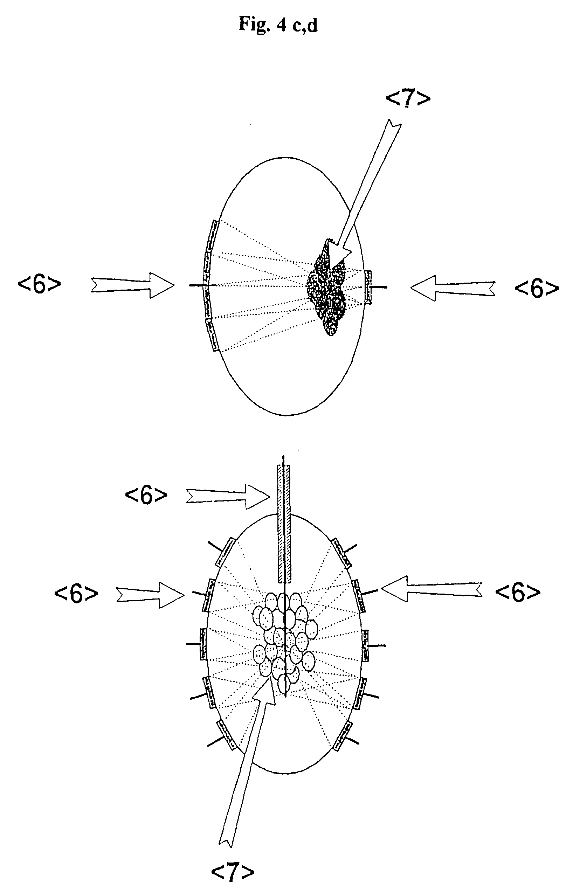

Figs. 4a-d show embodiments of electrode applicators 5 for external treatment of a patient with the electrodes 6 applied in a restricted region on the patient and in different configurations around the tissue region 7, for example a tumor 7, which is to be treated. Figs. 4a and 4b show how by crosswise application of the electric high voltage pulses to different combinations of two electrodes 6, the result will be achieved, as marked in the Figure by the electric field force lines, that the electric field passes through all parts of the tissue region 7.

Figs. 4c-d show how electrodes are designed with abutment surfaces of different sizes in order for the field lines to be focused to the desired treatment region. At the beginning of the treatment, the electric high voltage pulses have, for example, a voltage which is adjusted in accordance with the distance between the electrodes. The voltage is then adjusted in accordance with the relationship:

Voltage = (constant) x (the distance between the pairwise

electrodes).

The value of the constant is varied in response to the type of tissue and is, as a rule, selected at values between approx. 500-3000 V/cm.

Once the treatment has commenced, the control unit and impedance measurement unit described below regulate the output voltage of the pulse regulator to values which entail that the sought-for electric field passes through the tissue.

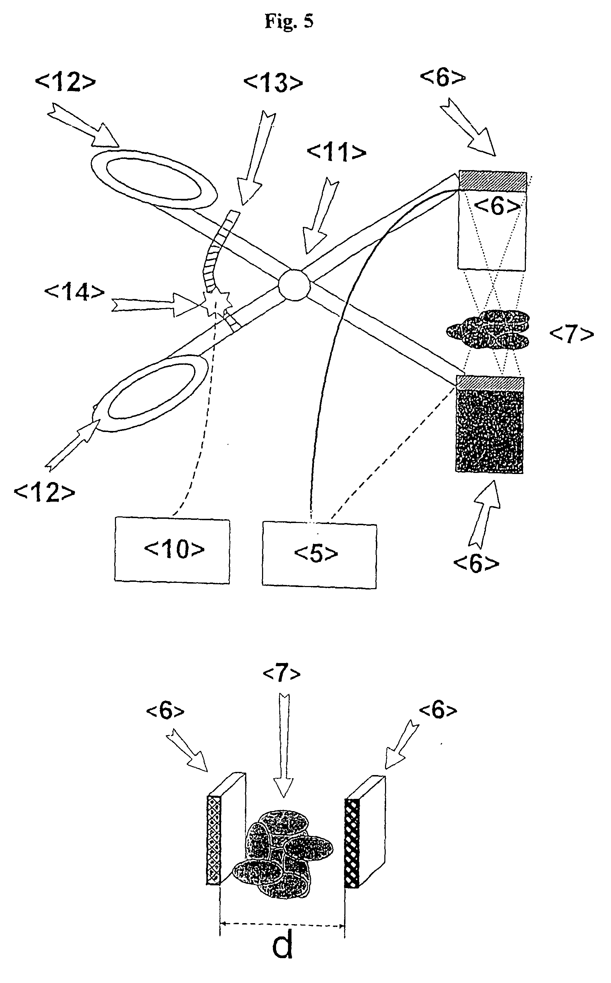

Fig. 5 shows one embodiment of an electrode applicator 5 for intraoperative treatment, and treatment of, for example, superficial tumor nodules 7. The electrode applicator has a scissors-like design and comprises two shanks 12 of electrically insulating material (e.g. teflon) which are movably interconnected to one another in a journal 11. The shanks are provided with a gripping lock 13. At one end of each shank 12, the shanks are provided with finger grips and at the other ends with electrodes 6 which grasp about the tumor nodules 7. The grip locks 13 fix the shanks 12 in the set position. The voltage of the electric high voltage pulses is adjusted in response to the size of the tumor 7 with the aid of a distance sensor 14 built into the electrode applicator and connected to the computer 10. The voltage is set at the beginning of the treatment, for example according to the relationship:

$$\text{Voltage} = \text{(constant)} \times \text{(the distance between the pairwise}$$

$$\text{electrodes).}$$

The value of the constant is adapted to the type of tumor and is, as a rule, selected within the range of approx. 500-3000 V/cm.

Once the treatment has commenced, the control unit and the impedance measurement unit described below regulate the output voltage of the pulse generator to values which entail that the sought-for electric field force passes through the tissue.

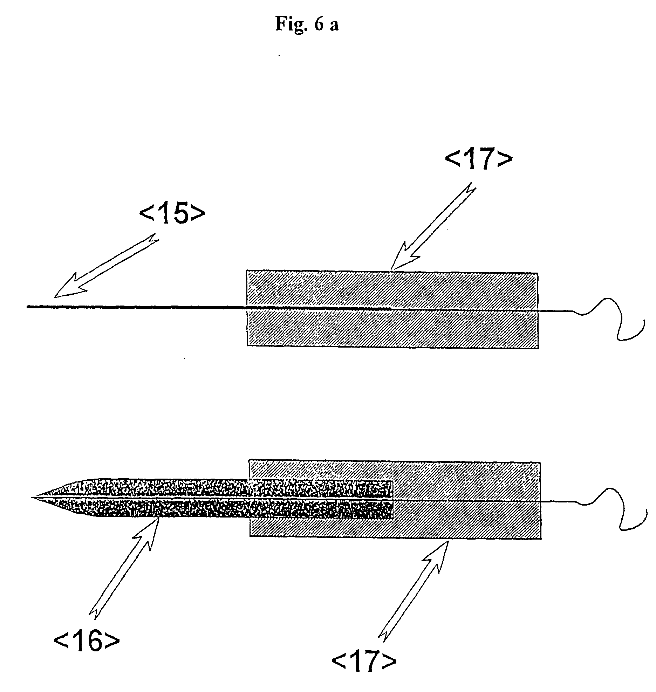

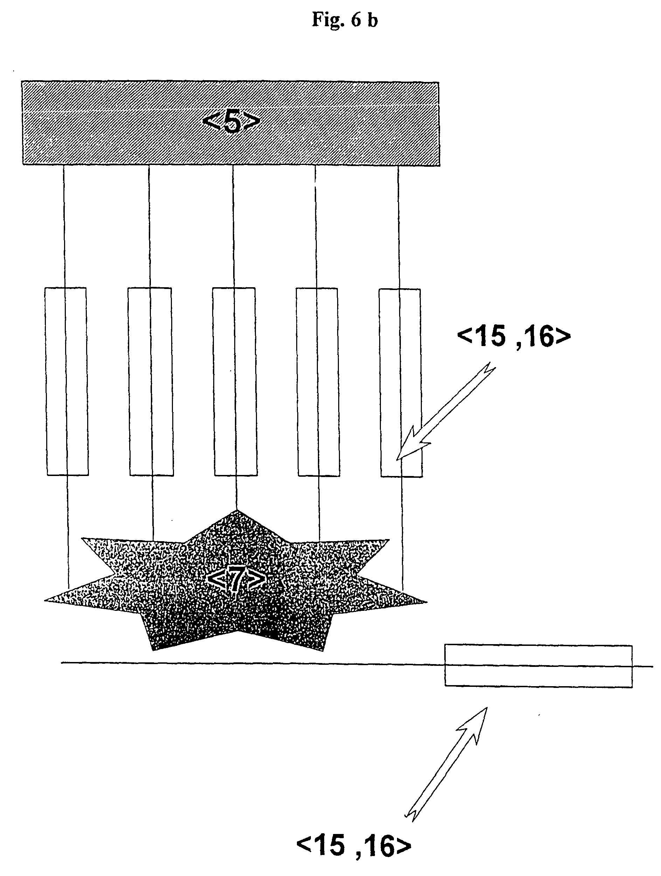

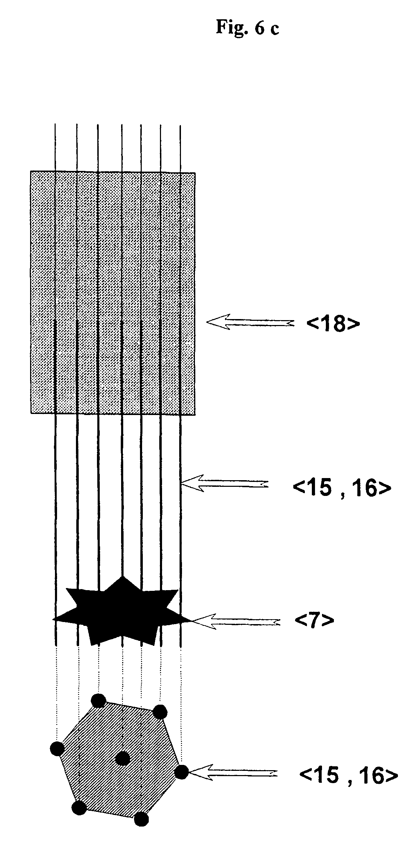

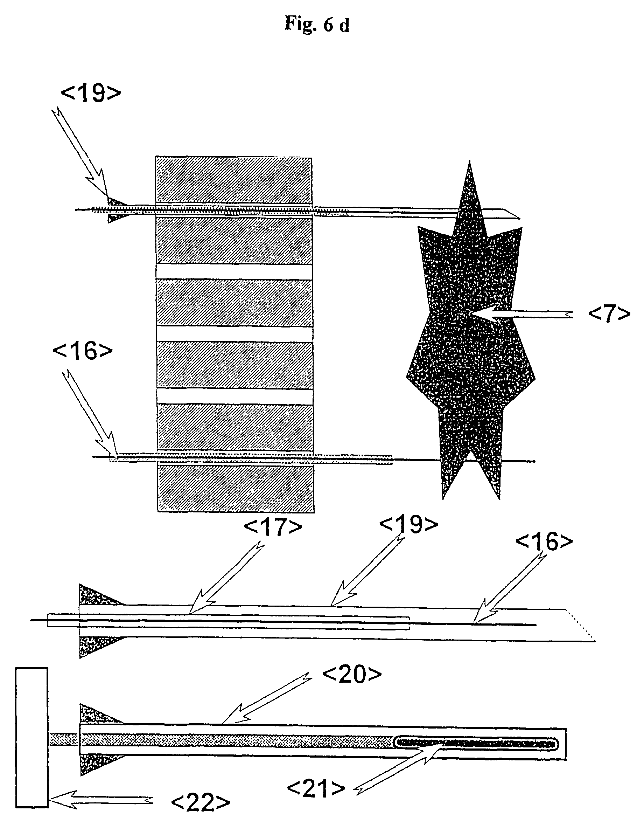

Figs. 6a-d show embodiments of electrodes 15,16 and a fixture 18 for the electrodes, where the electrodes and the fixture are suitable for use for interstitial treatment of both superficial and profound tissue. Fig. 6a shows the electrodes 15,16 in two different embodiments, namely in one embodiment in which the electrodes 15 are needle-shaped and in one embodiment in which the electrodes 16 are stiletto-shaped. Each one of the electrodes 15,16 is, in a portion 31 most proximal their one end, provided with an electric conductor 33 for connection to the high voltage generator 1. The above-mentioned portion is provided with an electrically insulating layer 17 or an electrically insulating sleeve 17 in which the electrode is inserted.

The electrodes are applied in different configurations in and about the tissue 7 or the organ 7 which is to be treated, either direct by free hand or with the aid of an electrode applicator (fixture) 18 provided with a hole. The electrode applicator is, as a rule, designed so as to be removed from the electrodes 15,16 once these have been applied on the patient. It will thereby be possible to allow the electrodes to remain in position in the patient to be used on several subsequent treatment occasions. Alternatively, the electrode applicator is removed together with the electrodes 15,16 after each treatment. Also in interstitial treatment, there are electrodes with surfaces of different sizes for controlling the extent of the electric fields.

Those parts of the electrodes 15,16 which are intended to be inserted into the patient to cover the extent of the tissue 7 which is to be treated are, for example, manufactured of stainless steel of a quality which agrees with or corresponds to that employed for injection syringes or are manufactured or coated with another tissue-friendly metal such as a noble metal, for example gold or platinum. The remaining portion of the electrodes forms an insulated portion 17 with input conductors 33 for the high voltage pulses. On the employment of flexible input conductors, the electrode is placed in a large cannula 19 which, after application of the electrode in the patient, is withdrawn, the electrodes remaining in position in the tissue.

In certain embodiments, the electrodes consist of radioactive metal (e.g. iridium-192, cobalt-60) or are surface coated with radioactive substances (e.g. iodine-125). In other embodiments, they are designed as tubes 20 of inert metal which are charged with radioactive material (e.g. $^{192}$Ir, $^{137}$Cs, $^{226}$Ra) which advantageously takes place by the employment of a so-called after loading device 22. The pulses have a voltage which, at the start of the treatment, for example are determined by the distance between the electrodes. The voltage is then set according to the relationship:

$$\text{Voltage} = \text{(constant)} \times \text{(the distance between pairwise electrodes).}$$

The value of the constant is selected in response to the type of tumor and, as a rule, selected within the range of approx. 500-3000 V/cm.

Once the treatment has commenced, the control unit and the impedance measurement unit described below regulate the output voltage of the pulse generator to values which entail that the sought-for electric field force passes through the tissue.

In those applications where treatment with electric fields is combined with radiation treatment from a radiation source which is located outside the treatment region, the electrodes in the treatment region are supplied with electric voltage pulses before, during or immediately after the radiation treatment.

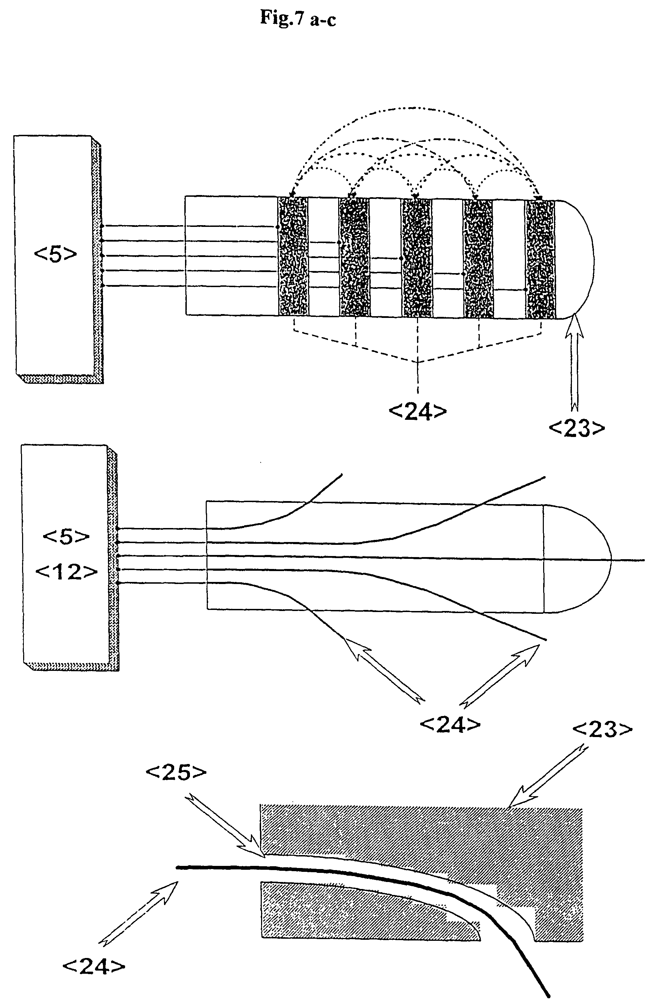

Figs. 7a-c show electrodes 24 for treating tissue accessible via, for example, major vessels, or via bodily cavities, for example respiratory tracts, urinary tracts and stomach-intestinal trait. The electrodes are disposed on the surface of a cylinder-like electrode applicator 23 of insulating material 17. In certain embodiments, the electrodes are designed such that they are introduced into the tissue through channels 25 in the applicator 23 operated by a remote control. As will be apparent from Fig. 7c, the channels 25 (according to the embodiment described in the preceding sentence) discharge in the circumferential surface of the electrode applicator, whereby the electrodes 24 are, on their displacement, guided into tissue surrounding the electrode applicator. In certain embodiments, the applicator is disposed to be supplied with radioactive preparations, whereby the applicator also forms a radiation device. The applicator is disposed to be supplied with the radioactive preparations manually or by means of an after loading device 22. The voltage of the electric high voltage pulses is adjusted during the treatment.

The field lines in Fig. 7a indicate the extent of the electric field lines in the tissue.

For intracavity treatment of tissue in different, irregularly shaped bodily cavities (e.g. oral cavity, respiratory tracts, oesophagus, stomach, uterus, bladder, ureter, rectum) electrode applicators 23 are applied as is apparent from Figs. 7a-c, particularly designed in response to the configuration of the cavity, with electrodes applied on the surface 24 or alternatively designed as needles which, through channels 25, are passed into the tissue by remote control. These applicators are suitable for use when treating, for example, lung cancer, liver tumors, renal tumors and tumors in the stomach-intestines with reduced absorbed dose for reducing side effects of the radiation treatment in normal tissue. Prostate cancer is treated with applicators applied via the rectum and the ureter. These applicators are, in certain embodiments, designed to be charged with radioactive sources or radioactive material 21, either manually or using an after loading device 22.

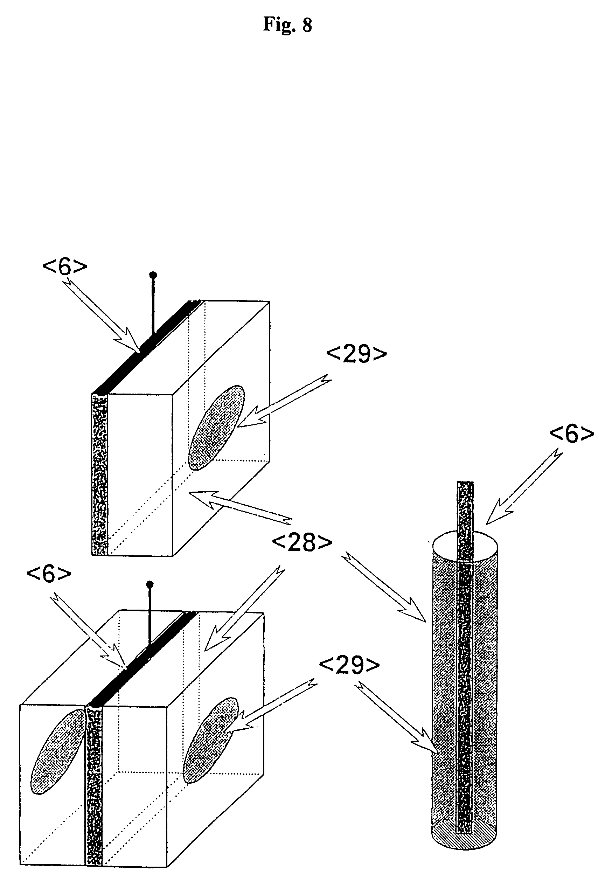

Fig. 8 shows an apparatus for combined treatment with antitumoral pharmaceutics where the electrode 6 is coated with a layer 28 of porous metal, glass, ceramics, inert plastic or other polymer which contains antitumoral pharmaceuticals 29 (e.g. bleomycin, platinol, taxol, monoclonal antibodies), genetic material (chromosomes, DNA) or radioactive substances (e.g. iodine 125, Auger-electron emitters) 29. This type of electrode is well suited for use in radiation therapy, since the high electric field increases the permeability of the tumor cell for the above mentioned substances and thereby increases the antitumoral effect.

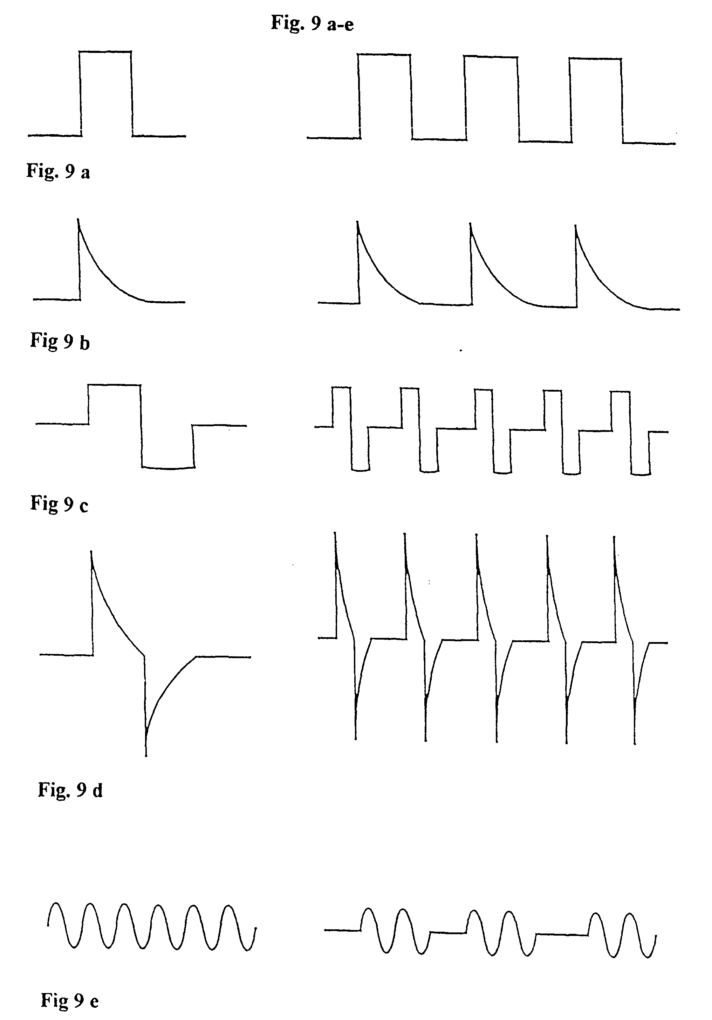

Figs. 9a-e show examples of pulse forms in the voltage pulses which are pairwise applied to the electrodes 6,15,16,24. In the Figures the height of the pulse represents the voltage between two electrodes. The width of the pulse represents the length of the pulse. The Figures 9a and 9c show examples of square pulses, Figs. 9b and 9d examples of pulses whose voltage fades with time, and Fig. 9e pulses of alternating voltage. Figs. 9c and 9d show voltage pulses where, analogous to that which applies in alternating voltage, the electrodes alternatingly have the highest voltage, whereby a corresponding change takes place of the electric field between the electrodes.

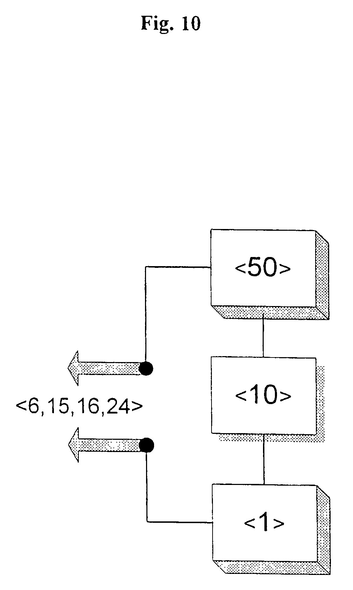

The above described electrodes 6,15,16,24, the voltage generator 1, the control and converter device 10, also previously designated the computer and an impedance measurement unit 50 are included in the block diagram shown in Fig. 10. The voltage generator, the computer, the electrodes and the impedance measurement unit are interconnected with one another by electric conductors for impressing voltage on the electrodes and for transferring signals. It will be obvious to a person skilled in the art that, in certain embodiments, at least a part of the signal connections are designed as wireless connections.

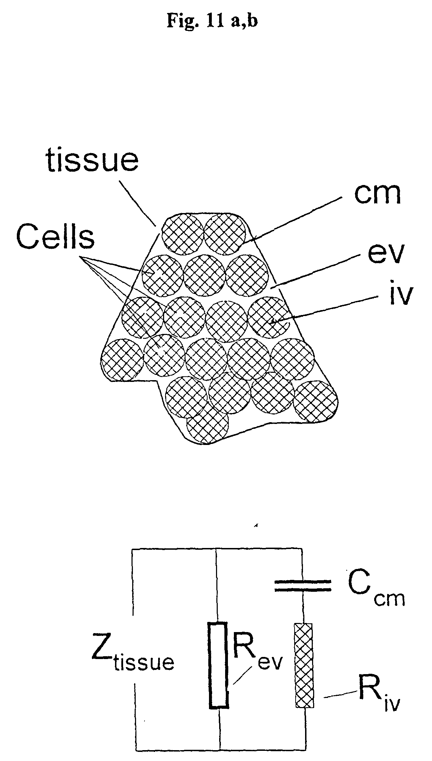

Fig. 11a shows the basic structure of living tissue, while Figure 11b shows an electric outline diagram for the electric structure of the tissue. The correspondences between the resistances and the capacitance in the electric diagram and in the tissue are apparent from the designations of the components and the continued description.

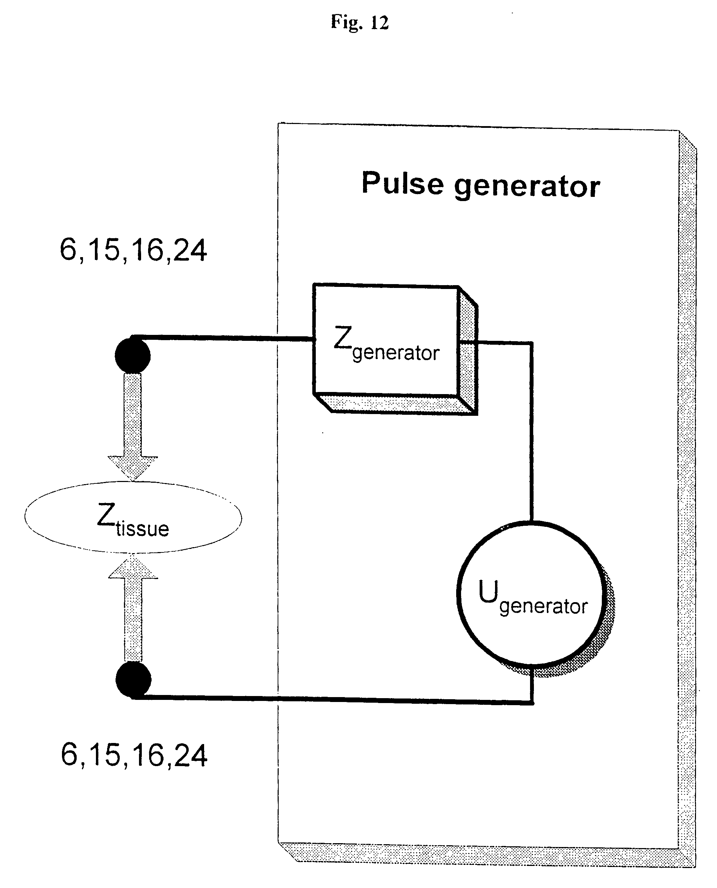

Fig. 12 shows the basic electric structure of a pulse generator 1, previously also designated high voltage generator. The Figure shows how the impedance of the tissue $Z_{tissue}$ via the electrodes 6,15,16,24 is connected in series to the inner impedance of the pulse generator $Z_{generator}$. Reference letter U relates to electromotive force (EMF) of the pulse generator.

It will be obvious to a person skilled in the art that the above described mechanical units, in certain embodiments of the present invention, form mutually separate mechanical units which are interconnected with each other by means of electric conductors and signal connections, while, in other embodiments, some or all of these units, with the exception of the electrode applicator and the electrodes, form a mechanical unit which is co-ordinated with the voltage generator, the impedance measurement unit or the computer.

As will have been apparent from the foregoing description, the present invention relates to an apparatus for high voltage impulse therapy (HVIT) with detection of the treatment effect. The apparatus includes an impedance measurement unit which, on treatment of tissue or organs, is employed for measuring the electrical impedance of the tissue. The impedance measurement unit is disposed to measure the impedance of the tissue at, at least, one frequency. Normally, the impedance measurement unit is disposed to measure the impedance of the tissue within a frequency range, e.g. within the range of 10 Hz to 10 Mhz. With the aid of a mathematical algorithm, a test magnitude is calculated whose value is a measurement of the treatment effect.

The voltage across the tissue will be in accordance with that shown in Fig. 12:

$$U_{tissue} = U_{generator} * Z_{tissue} / (Z_{tissue} + Z_{generator})$$

The impedance of the tissue varies extremely, depending upon the cell structure and build up of the tissue, the nature of the surrounding tissue and the quantity of bodily liquids which are found in and around the treated region. Since the output impedance of the generator is not slight in relation to the impedance of the tissue, the output voltage will vary greatly depending upon where and how the applicator is placed. It has proved, in practical experiments, that even if an applicator is placed at the same point, marked with a colour on the body, the impedance will vary greatly from time to time, depending upon minor differences in placing and contact impedance, as well as differences in fluid quantity and the nature of the tissue.

In order to be able to predict the actual pulse voltage from the pulse generator, the impedance of the tissue must be known at any time. Only if the output voltage from the generator is adjusted on the basis of the generator's output impedance and the impedance of the relevant tissue will it be possible to achieve a predictable and constant effect. According to the present invention, the apparatus includes means for measuring the impedance of the treated tissue and means for employing this information for controlling the output voltage of the pulse generator such that the desired field force is always achieved in the tissue.

Fig. 10 illustrates such a system. A control unit is included in the apparatus and measures, with the aid of the impedance measurement unit, the impedance of the tissue. The control unit adjusts the output voltage from the generator so that the desired field is achieved.

In the control unit, which, for example, is a PC, the desired field is set whereafter the control unit measures the impedance in the tissue and calculates the requisite pulse voltage from the generator. When a pulse is subsequently applied, the field will always be constant, since the control unit always measures and adjusts the voltage from the generator before the pulse is generated.

With the system in Fig. 10 the sought-for effect will be achieved, e.g. to maintain a constant output voltage from the pulse generator independently of the impedance in the tissue. It also proves that a system according to Fig. 10 is excellent for measuring and assessing the treatment result achieved in HVIT. By measuring impedance and carrying out analysis of impedance change in the tissue after a pulse has been applied, the documentary support is given for assessing when the treatment is completed and no more pulses are needed or give a further positive effect. This method builds on the tissue model shown in Fig. 11a,b.

The impedance in tissue substantially consists of three components, the resistance in the extra cellular fluid, the resistance in the intra cellular fluid and the capacitance which is formed between the D.C. insulating effect of the cell membrane. In the model, we have combined the impedance effect of the cell core with the resistance in the intracellular fluid. At low frequencies, only current will flow through the extra cellular liquid and the impedance is determined substantially by $R_{ev}$. At medium-high frequencies, the capacitance of the cell membrane Ccm together with the resistance of the intracellular liquid, $R_{iv}$, will begin to effect the impedance. At high frequencies, substantially the components $R_{ev}$ and $R_{iv}$ will effect the impedance of the tissue. Thus, the result will be a frequency dependence in the impedance of the tissue which is largely dependent on the thickness of the cell membrane and the formation of the cells. At low frequencies, the impedance is approximately $R_{ev}$ and at high frequencies $R_{ev}//R_{iv}$. The symbol // is employed to indicate that Rev is connected in parallel with $R_{iv}$.

$$Z_{tissue} = R_{ev} // ( R_{iv} + C_{cm})$$

Since the treatment with electric fields is intended to render the cell membrane permeable or to wholly destroy it, a clear indication will be obtained by measuring the change in $C_{cm}$ as to whether the treatment is completed or not. When all cell membranes in the tissue are destroyed, no change of Ccm will take place any longer and the tissue is ready-treated.

Table 1 below illustrates a compilation of impedance measurements taken during the treatment of rats with tumors.

Table 1

| Measured tissue impedance in ohm in rats with tumor | | | | | |
|---|---|---|---|---|---|
| Frequency/Pulses | 0 pulses | 16 pulses | 32 pulses | 48 pulses | 64 pulses |
| 10 Hz | 232.24 | 160.12 | 160.36 | 172.53 | 179.3 |
| 15 Hz | 229.42 | 157.76 | 151.48 | 163.37 | 159.61 |
| 20 Hz | 200.28 | 145.46 | 138.84 | 148.89 | 141.78 |
| 30 Hz | 173.9 | 134.11 | 127.56 | 132.16 | 125.87 |
| 50 Hz | 153.7 | 122.75 | 116.44 | 120 | 112.29 |
| 70 Hz | 144.46 | 116.39 | 110.38 | 136.26 | 105.58 |
| 100 Hz | 137.64 | 110.69 | 105.13 | 105.47 | 100.31 |
| 150 Hz | 130.68 | 104.86 | 99.79 | 99.71 | 95.35 |
| 200 Hz | 125.81 | 100.97 | 96.31 | 96.23 | 92.26 |
| 300 Hz | 120.3 | 96.27 | 92.06 | 92.19 | 88.73 |
| 500 Hz | 113.96 | 91.09 | 87.49 | 87.84 | 84.91 |
| 700 Hz | 109.83 | 87.88 | 84.68 | 85.16 | 82.6 |
| 1000 Hz | 105.88 | 85.03 | 82.2 | 83.03 | 80.62 |
| 1500 Hz | 101.99 | 82.12 | 79.71 | 81.84 | 78.59 |
| 2000 Hz | 99.34 | 80.27 | 78.02 | 79.54 | 77.69 |
| 3000 Hz | 96.12 | 77.98 | 76.06 | 77.18 | 75.72 |
| 5000 Hz | 92.28 | 75.4 | 73.81 | 74.85 | 73.71 |
| 7000 Hz | 89.72 | 73.85 | 72.41 | 73.86 | 72.54 |
| 10000 Hz | 87.38 | 72.45 | 71.14 | 73.52 | 71.43 |
| 15000 Hz | 84.91 | 70.91 | 69.71 | 72.53 | 70.15 |
| 20000 Hz | 83.18 | 69.75 | 68.62 | 71.51 | 69.17 |
| 30000 Hz | 80.8 | 68.23 | 67.14 | 69.81 | 67.8 |
| 50000 Hz | 77.73 | 66.26 | 65.28 | 68.24 | 65.97 |
| 70000 Hz | 75.62 | 64.79 | 63.9 | 66.67 | 64.65 |
| 100000 Hz | 73.01 | 63.01 | 62.11 | 64.62 | 62.93 |
| 150000 Hz | 70.42 | 61.05 | 60.3 | 64.06 | 61.19 |
| 200000 Hz | 68.3 | 59.37 | 58.76 | 61.93 | 59.65 |

It will be apparent from Table 1 that the impedance reduces at low and medium-high frequencies after treatment with pulses. The reduction principally takes place after the introductory 16 pulses and the change rapidly fades thereafter. Thus, the rat is substantially ready-treated already after the first 16 pulses and further treatment after 32 or 48 pulses gives no major change in $C_{cm.}$. The measurement data in Table 1 indicates that the treatment is completed after 32 pulses. In order to confirm this assessment, the measured measurement values have been taken and treated as described below.

Table 2 shows the impedance change in per cent at different frequencies after the electric fields generated by 16 voltage pulses have passed through the tissue. In the Table, the change of the impedance is given in per cent which occurred each time when a series of electric fields generated by the voltage pulses has passed through the tissue.

Table 2

| Impedance change in per cent after treatment with 16 pulses at a time | | | | |
|---|---|---|---|---|
| **Frequency/Pulses** | **16 pulses** | **32 pulses** | **48 pulses** | **64 pulses** |
| 10 Hz | -31.05408 | 0.1033414 | 5.2402687 | 2.9150878 |
| 15 Hz | -31.23529 | -2.737338 | 5.1826345 | -1.638916 |
| 20 Hz | -27.37168 | -3.305372 | 5.0179748 | -3.55003 |
| 30 Hz | -22.88097 | -3.766532 | 2.6451984 | -3.617021 |
| 50 Hz | -20.13663 | -4.1054 | 2.3162004 | -5.016265 |
| 70 Hz | -19.43098 | -4.160321 | 17.914994 | -21.23771 |
| 100 Hz | -19.58006 | -4.039523 | 0.2470212 | -3.74891 |
| 150 Hz | -19.75819 | -3.879706 | -0.061218 | -3.336394 |
| 200 Hz | -19.74406 | -3.703998 | -0.063588 | -3.155552 |
| 300 Hz | -19.97506 | -3.499584 | 0.1080632 | -2.876143 |
| 500 Hz | -20.06845 | -3.159003 | 0.3071253 | -2.571078 |
| 700 Hz | -19.98543 | -2.913594 | 0.4370391 | -2.330875 |
| 1000 Hz | -19.6921 | -2.672837 | 0.7839063 | -2.276162 |
| 1500 Hz | -19.4823 | -2.362977 | 2.08844 | -3.186587 |
| 2000 Hz | -19.1967 | -2.264949 | 1.5300987 | -1.862291 |
| 3000 Hz | -18.87224 | -1.997503 | 1.1652102 | -1.518935 |
| 5000 Hz | -18.29215 | -1.723017 | 1.1270048 | -1.235371 |
| 7000 Hz | -17.68836 | -1.604993 | 1.6161391 | -1.471244 |
| 10000 Hz | -17.08629 | -1.499199 | 2.7237354 | -2.391852 |
| 15000 Hz | -16.48805 | -1.413261 | 3.3211636 | -2.802968 |
| 20000 Hz | -16.14571 | -1.3585 | 3.4743929 | -2.813176 |
| 30000 Hz | -15.55693 | -1.34901 | 3.3044554 | -2.487624 |
| 50000 Hz | -14.75621 | -1.260774 | 3.8080535 | -2.920365 |
| 70000 Hz | -14.32161 | -1.176937 | 3.6630521 | -2.671251 |
| 100000 Hz | -13.69675 | -1.232708 | 3.4378852 | -2.314751 |
| 150000 Hz | -13.30588 | -1.065038 | 5.3393922 | -4.075547 |
| 200000 Hz | -13.07467 | -0.893119 | 4.6412884 | -3.338214 |

The heading of the Table discloses the accumulated number of pulses of electric fields which have passed through the tissue. On each treatment occasion, a series of 16 pulses is passed through the tissue. That disclosed in this paragraph for the table heading in Table 2 also applies to the table headings for Tables 3 and 4 used below.

It will be apparent from Table 2, in the same manner as Table 1, that the treatment may be discontinued after 32 pulses, since the impedance change fades dramatically. Table 3 below shows the mean value of the impedance change after different numbers of pulses. The mean value is formed from all measurement frequencies between 10 Hz and 200 kHz. In Table 3, it is clearly seen that the largest impedance change takes place after the first 16 pulses and only a slight change takes place on further treatment.

Table 3

| Progressive change in per cent of impedance value at frequencies between 10 Hz-200 kHz | | | |
|---|---|---|---|
| 16 pulses | 32 pulses | 48 pulses | 64 pulses |
| -19.9568 | -2.424687 | 3.1275358 | -3.366544 |

In Table 4, in mean value formation, frequencies below 100 Hz and frequencies over 10 kHz have been deleted. By deleting the lowest frequencies from the mean value, this prevents incorrect impedance values because of disturbance from the motorsystem of the body from influencing the result. The highest frequencies are deleted since the impedance change at these high frequencies is less when Ccm is changed and therefore does not contribute to an improved picture of the treatment result.

Table 4

| Progressive change in per cent of impedance values at frequencies between 100 Hz-10 kHz | | | |
|---|---|---|---|
| 16 pulses | 32 pulses | 48 pulses | 64 pulses |
| -20.78512 | -2.943407 | 1.0007481 | -2.663449 |

By allowing the control unit in Fig. 10 mathematically to treat and present the measured treatment result as described above, there will be obtained an apparatus which satisfies the wishes of controlling, in the treatment, the strength of the electric field in order to obtain a basis for discontinuing the treatment at the correct moment and for being able to interpret the direct outcome of the treatment with the electric field.

From the foregoing description, it will be apparent that, in a very simple application of the present invention, the impedance of the tissue is determined at only one frequency. In such instance, a medium-high frequency, e.g. 15 kHz is selected. The inner impedance of the pulse generator is entered in the computer as a fixed value, whereby the impedance of the tissue is determined by a mathematical operation corresponding to that described above. In applications of the present invention, use is however made as a rule of many frequencies in order to eliminate the risks of any possible disruptions which may affect the measurement results.

The system illustrated in Fig. 10 includes means for adjusting the pulse voltage and its frequency content so that the electric field in the treated tissue is always constant regardless of impedance or resistance changes in the tissue. Such means also give a basis for assessing the achieved treatment effect in that it is of a structure which makes it possible to present, for example readily understandable values and graphs which, by mathematical operations, have been extracted from measured impedance or resistance data.

On practical application of the present invention in the embodiment where a radiation transmitter is employed, the radiation transmitter and the electrodes, in certain applications together with the electrode applicator and impedance measurement unit, together form a cohesive mechanical unit. This is of a design which makes it possible, in a restricted region of a human or an animal, to apply both the radiation transmitter and the electrodes in positions where the ionizing radiation is directed at the tissue which is being treated and where the electrodes are in positions in which electric fields between them pass through the tissue. In other embodiments, such means constitute separate parts which, together and where applicable temporarily, or for a lengthy period of time, form a system of devices of a composition corresponding to that described above for the apparatus 40.

The above detailed description has referred to but a limited number of embodiments of the present invention, but a person skilled in the art will readily perceive that the present invention encompasses a large number of embodiments without departing from the scope of the appended claims.

**Claims**

1.   An apparatus (40) for the therapy of tissue or an organ, comprising:

  -   a plurality of electrodes (6, 15, 16, 24) adapted for placement within a region of the tissue or the organ,
  -   a voltage generator (1) arranged to generate one or more voltage pulses to said plurality of electrodes (6, 15, 16, 24),
  -   an impedance measuring unit (50) arranged between the plurality of electrodes (6, 15, 16, 24) for measuring the impedance of the treated tissue or organ between the electrodes, and
  -   a control unit (10) arranged between said voltage generator (1) and said impedance measuring unit (50), and arranged to receive impedance measurements from the impedance measuring unit (50), the control unit (10) being adapted to control, in dependence of said impedance measurements, the size, number, configuration and/or duration of the voltage pulses, **characterized in that** the control unit (10) controls the voltage generator (1) to adjust the amplitude of the pulses so that a predetermined electric field in said region is produced.

2. The apparatus as claimed in Claim 1, wherein the control unit (10) includes a VDU (10a); wherein the control unit is disposed, prior to the start of the generation by the voltage generator (1) of a pulse or chain of pulses, to show on the VDU (10a) the form of the pulse or chain of pulses calculated by the control unit and wherein means are included in said control unit for manual or automatic acceptance of said calculated formation.

3. The apparatus as claimed in any of the preceding Claims, wherein the apparatus includes means (34) for supplying therapeutic substances, genetic material and/or ionizing radiation to said restricted region of a human or of an animal.

4. The apparatus as claimed in any of the preceding Claims, wherein the apparatus includes sensors (8) for detecting electric fields formed by the electrodes (6, 15, 16, 24); and that the sensors are connected to the control unit (10), said unit (10) being able to calculate the electric field and the electromotive force in different parts of the treatment region (7) from sensor signals.

5. The apparatus as claimed in any of the preceding Claims, wherein the electrodes (6) are disposed to be excited alternatively and only two at a time.

6. The apparatus as claimed in any of the preceding Claims, wherein the apparatus includes sensors (14) for detecting the distance between the electrodes (6) in each pair of excited electrodes; and that said control unit(10) includes means for adjusting the voltage between the electrodes (6) in each pair of excited electrodes based on the distance between the electrodes.

7. The apparatus as claimed in any of the preceding Claims, wherein the control unit (10) analyses the impedance change in the tissue for assessing when the treatment is completed and no more pulses are needed.

8. The apparatus as claimed in any of the preceding Claims, wherein the control unit (10) controls the voltage generator (1), before the pulses are generated, to adjust the amplitude of the pulses so that a predetermined electric field in said region is obtained.

9. The apparatus as claimed in any of the preceding Claims, wherein the one or more voltage pulse have a pulse length of 0.1 to 200 ms.

10. The apparatus as claimed in any of the preceding Claims, wherein the impedance measuring unit (50) measures at frequencies within the range of 10 Hz to 10 MHz.

11. The apparatus as claimed in any of the preceding Claims, wherein the voltage generator (1) is disposed to emit modulated a.c. voltage of a frequency within the range of 40 Hz to 2 MHz, preferably within 40 Hz - 100 kHz.

12. The apparatus as claimed in any of the preceding Claims, wherein the impedance measuring unit (50) measures at frequencies within the range of 10 Hz - 200 kHz.

13. The apparatus as claimed in any of the preceding Claims, wherein the impedance measuring unit (50) measures at frequencies within the range of 100 Hz to 10 kHz.

14. The apparatus as claimed in any of the preceding Claims, wherein one or more voltage pulses are set to a repetition frequency of 0.1 to 10000 per second.

15. The apparatus as claimed in any of the preceding Claims, wherein amplitude of the one or more pulses is 50 to 6000 V.

16. The apparatus as claimed in any of the preceding Claims, wherein the control unit (10) is a computer or a microprocessor.

**EP 1 067 985 B1**

17. The apparatus as claimed in any of the preceding Claims, wherein said electrodes (6, 15, 16, 24) are needles (15) or stilettos (16).

18. The apparatus as claimed in any of the preceding Claims, wherein said electrodes (6, 15, 16, 24) are surrounded by an electrically insulating layer (17).

19. The apparatus as claimed in any of the preceding Claims, comprising an electrode applicator (5, 23) arranged to at least temporarily fixate the electrodes (6, 15, 16, 24) prior to the placing of the electrodes in said region.

20. The apparatus sis claimed in Claim 19, wherein said electrode applicator (23) includes a fixture (18) provided with a number of holes and arranged to place the electrodes (6, 15, 16, 24) in a desired pattern.


**Patentansprüche**

1. Vorrichtung (40) zur Therapie von Gewebe oder eines Organs, mit:

   - mehreren Elektroden (6, 15, 16, 24), die in einem Bereich des Gewebes oder des Organs angeordnet werden können,
   - einem Spannungsgenerator (1), der so beschaffen ist, dass er einen oder mehrere Spannungsimpulse für die mehreren Elektroden (6, 15, 16, 24) erzeugt,
   - einer Impedanzmesseinheit (50), die zwischen den mehreren Elektroden (6, 15, 16, 24) angeordnet ist, um die Impedanz des behandelten Gewebes oder Organs zwischen den Elektroden zu messen, und
   - einer Steuereinheit (10), die zwischen dem Spannungsgenerator (1) und der Impedanzmesseinheit (50) angeordnet und so beschaffen ist, dass sie Impedanzmesswerte von der Impedanzmesseinheit (50) empfängt, wobei die Steuereinheit (10) so beschaffen ist, dass sie in Abhängigkeit von den Impedanzmesswerten die Größe, die Anzahl, die Konfiguration und/oder die Dauer der Spannungsimpulse steuert,

     **dadurch gekennzeichnet, dass** die Steuereinheit (10) den Spannungsgenerator (1) so steuert, dass er die Amplitude der Impulse in der Weise einstellt, dass in dem Bereich ein vorgegebenes elektrisches Feld erzeugt wird.

2. Vorrichtung nach Anspruch 1, bei der die Steuereinheit (10) eine VDU (10a) enthält; wobei die Steuereinheit so angeordnet ist, dass sie vor dem Beginn der Erzeugung eines Impulses oder einer Impulskette durch den Spannungsgenerator (1) auf der VDU (10a) die Form des Impulses oder der Impulskette, die durch die Steuereinheit berechnet wird, zeigt, wobei in der Steuereinheit Mittel enthalten sind, um die berechnete Formung manuell oder automatisch zu akzeptieren.

3. Vorrichtung nach einem der vorhergehenden Ansprüche, wobei die Vorrichtung Mittel (34) für die Zuführung von therapeutischen Substanzen und/oder kinetischem Material und/oder ionisierender Strahlung zu dem begrenzten Bereich eines Menschen oder eines Tiers enthält.

4. Vorrichtung nach einem der vorhergehenden Ansprüche, wobei die Vorrichtung Sensoren (8) zum Erfassen elektrischer Felder, die durch die Elektroden (6, 15, 16, 24) erzeugt werden, enthält; wobei die Sensoren mit der Steuereinheit (10) Verbundes sind, wobei die Steuereinheit (10) das elektrische Feld und die elektromotorische Kraft in verschiedenen Teilen des Behandlungsbereichs (7) anhand von Sensorsignalen berechnen kann.

5. Vorrichtung nach einem der vorhergehenden Ansprüche, bei der die Elektroden (6) so angeordnet sind, dass sie abwechselnd erregt werden und dass zu einem Zeitpunkt nur zwei von ihnen erregt werden.

6. Vorrichtung nach einem der vorhergehenden Ansprüche, wobei die Vorrichtung Sensoren (14) zum Erfassen des Abstandes zwischen den Elektroden (6) in jedem Paar erregter Elektroden umfasst; und wobei die Steuereinheit (10) Mittel enthält, um die Spannung zwischen den Elektroden (6) in jedem Paar erregter Elektroden anhand des Abstandes zwischen den Elektroden einzustellen.

7. Vorrichtung nach einem der vorhergehenden Ansprüche, wobei die Steuereinheit (10) die Impedanz Veränderung in der Gewebe analysiert, um wenn der Behandlung vertig ist und keine Pulsen notwendig ist, zu evaluieren.

8. Vorrichtung nach einem der vorhergehenden Ansprüche, wobei die Steuereinheit (10)den Spannungsgenerator

**12**

(1) so steuert, dass er während die Impulse die Amplitude der Impulse in der Weise einstellt, dass ein vorgegenenes elektrisches Feld erzeugt wird.

**9.** Vorrichtung nach einem der vorhergehenden Ansprüche, bei der der eine oder die mehreren Spannungsimpulse eine Impulslänge von 0,1 bis 200 ms haben.

**10.** Vorrichtung nach einem der vorhergehenden Ansprüche, bei der die Impedanzmesseinheit (50) bei Frequenzen im Bereich von 10 Hz bis 10 MHz misst.

**11.** Vorrichtung nach einem der vorhergehenden Ansprüche, bei der der Spannungsgenerator (1) so angeordnet ist, dass er eine modulierte Wechselspaanung mit einer Frequenz im Bereich von 40 Hz bis 2 MHz und vorzugsweise im Bereich von 40 Hz-100 kHz emittiert.

**12.** Vorrichtung nach einem der vorhergehenden Ansprüche, bei der die Impedanzmesseinheit (50) bei Frequenzen im Bereich von 10 Hz-200 kHz misst.

**13.** Vorrichtung nach einem der vorhergehenden Ansprüche, bei der die Impedanzmesseinheit (50) bei Frequenzen im Bereich von 100 Hz bis 10 kHz misst.

**14.** Vorrichtung nach einem der vorhergehenden Ansprüche, bei der der eine oder die mehreren Spannungsimpulse eine Wiederholfrequenz von 0,1 bis 10000 pro Sekunde gesetzt sind.

**15.** Vorrichtung nach einem der vorhergehenden Ansprüche, bei der die Amplitude des einen oder der mehreren Impulse 50 bis 6000 V beträgt.

**16.** Vorrichtung nach einem der vorhergehenden Ansprüche, bei der die Steuereinheit (10) ein Computer oder ein Mikroprozessor ist.

**17.** Vorrichtung nach einem der vorhergehenden Ansprüche, bei der die Elektroden (6, 15, 16, 24) Nadeln (15) oder Stilette (16) sind.

**18.** Vorrichtung nach einem der vorhergehenden Ansprüche, bei der die Elektroden (6, 15, 16, 24) von einer elektrisch isolierenden Schicht (17) umgeben sind.

**19.** Vorrichtung nach einem der vorhergehenden Ansprüche, die einen Elektrodenapplikator (5, 23) umfasst, der so beschaffen ist, dass er die Elektroden (6, 15, 16, 24) vor der Anbringung der Elektroden in dem Bereich wenigstens vorläufig fixiert.

**20.** Vorrichtung nach Anspruch 17, bei der der Elektrodenapplikator (23) eine Befestigungseinrichtung (18) aufweist, die mit mehreren Löchern versehen und so beschaffen ist, dass die Elektroden (6, 15, 16, 24) in einem gewünschten Muster angeordnet werden können.

**Revendications**

**1.** Appareil (40) permettant la thérapie de tissu ou d'un organe, comprenant :

- une pluralité d'électrodes (6, 15, 16, 24) adaptées pour être placées à l'intérieur d'une zone du tissu ou de l'organe,

- un générateur de tension (1) agencé pour produire une ou plusieurs impulsions de tension sur ladite pluralité d'électrodes (6, 15, 16, 24)

- une unité de mesure d'impédance (50) agencée entre la pluralité d'électrodes (6, 15, 16, 24) pour mesurer l'impédance du tissu ou de l'organe traité entre les électrodes, et

- une unité de commande (10) agencée entre ledit générateur de tension (1) et ladite unité de mesure d'impédance (50), et agencée pour recevoir des mesures d'impédance de l'unité de mesure d'impédance (50), l'unité de commande (10) étant adaptée pour commander, en fonction desdites mesures d'impédance, la taille, le nombre, la configuration et/ou la durée des impulsions de tension, **caractérisé en ce que** l'unité de commande (10) commande le générateur de tension (1) pour ajuster l'amplitude des impulsions de sorte qu'un champ électrique prédéterminé dans ladite région soit produit.

2. Appareil selon la revendication 1, dans lequel l'unité de commande (10) comprend un écran de visualisation (10a) ; dans lequel l'unité de commande est disposée, avant le début de la production par le générateur de tension (1) d'une impulsion ou d'une chaîne d'impulsions, pour montrer sur l'écran de visualisation (10a) la forme de l'impulsion ou de la chaîne d'impulsions calculée par l'unité de commande, et dans lequel des moyens sont compris dans ladite unité de commande pour l'acceptation manuelle ou automatique de ladite formation calculée.

3. Appareil selon l'une quelconque des revendications précédentes, dans lequel l'appareil comprend des moyens (34) pour fournir des substances thérapeutiques, du matériel génétique et/ou un rayonnement ionisant à ladite région restreinte d'un être humain ou d'un animal.

4. Appareil selon l'une quelconque des revendications précédentes, dans lequel l'appareil comprend des capteurs (8) pour détecter des champs électriques formés par les électrodes (6, 15, 16, 24) ; et les capteurs sont reliés à l'unité de commande (10), ladite unité de commande (10) étant adaptée pour calculer le champ électrique et la force électromotrice dans différentes parties de la zone de traitement (7) à partir des signaux du capteur.

5. Appareil selon l'une quelconque des revendications précédentes, dans lequel les électrodes (6) sont disposées pour être excitées en alternance et uniquement deux à la fois.

6. Appareil selon l'une quelconque des revendications précédentes, dans lequel l'appareil comprend des capteurs (14) pour détecter la distance entre les électrodes (6) dans chaque paire d'électrodes excitées ; et ladite unité de commande (10) comprend des moyens pour ajuster la tension entre les électrodes (6) dans chaque paire d'électrodes excitées sur la base de la distance entre les électrodes.

7. Appareil selon l'une quelconque des revendications précédentes, dans lequel l'unité de commande (10) analyse impédance changement dans les tissu pour évaluer quand le traitement est fini et aucune impulsions soit nécessaire.

8. Appareil selon l'une quelconque des revendications précédentes, dans lequel l'unité de commande (10) commande le générateur de tension (1), avant les impulsions soit produit, pour ajuster l'amplitude des impulsions, de sorte qu'un champ électrique prédéterminé dans ladite région soit produit.

9. Appareil selon l'une quelconque des revendications précédentes, dans lequel une ou plusieurs impulsions de tension ont une longueur d'impulsion de 0,1 à 200 ms.

10. Appareil selon l'une quelconque des revendications précédentes, dans lequel l'unité de mesure d'impédance (50) mesure à des fréquences comprises dans la plage allant de 10 Hz à 10 MHz.

11. Appareil selon l'une quelconque des revendications précédentes, dans lequel le générateur de tension (1) est disposé pour émettre une tension de courant alternatif modulée d'une fréquence comprise dans la plage allant de 40 Hz à 2 MHz, de préférence, 40 Hz à 100 kHz.

12. Appareil selon l'une quelconque des revendications précédentes, dans lequel l'unité de mesure d'impédance (50) mesure à des fréquences comprises dans la plage allant de 10 Hz à 200 kHz.

13. Appareil selon l'une quelconque des revendications précédentes, dans lequel l'unité de mesure d'impédance (50) mesure à des fréquences comprises dans la plage allant de 100 Hz à 10 kHz.

14. Appareil selon l'une quelconque des revendications précédentes, dans lequel une ou plusieurs impulsions de tension sont fixées à une fréquence de répétition de 0,1 à 10000 par seconde.

15. Appareil selon l'une quelconque des revendications précédentes, dans lequel l'amplitude d'une ou de plusieurs impulsions est de 50 à 6000 V.

16. Appareil selon l'une quelconque des revendications précédentes, dans lequel l'unité de commande (10) est un ordinateur ou un microprocesseur.

**17.** Appareil selon l'une quelconque des revendications précédentes, dans lequel lesdites électrodes (6, 15, 16, 24) sont des aiguilles (15) ou des stylets (16).

**18.** Appareil selon l'une quelconque des revendications précédentes, dans lequel lesdites électrodes (6, 15, 16, 24) sont entourées d'une couche d'isolation électrique (17).

**19.** Appareil selon l'une quelconque des revendications précédentes, comprenant un applicateur d'électrodes (5, 23) agencé pour fixer les électrodes (6, 15, 16, 24) au moins temporairement avant le placement des électrodes dans ladite zone.

**20.** Appareil selon la revendication 19, dans lequel ledit applicateur d'électrodes (23) comprend un dispositif de fixation (18) pourvu d'un certain nombre de trous et agencé pour placer les électrodes (6, 15, 16, 24) dans un modèle svuhaité.

## Fig. 1

Fig. 2

Fig. 3

## Fig. 4 a,b

## Fig. 4 c,d

<7>

<6>

<6>

<6>

<6>

<6>

<6>

<7>

Fig. 5

Fig. 6 a

**Fig. 6 b**

<5>

<15 ,16>

<7>

<15 ,16>

**Fig. 6 c**

<18>

<15 , 16>

<7>

<15 , 16>

Fig. 6 d

## Fig.7 a-c

Fig. 8

**Fig. 9 a-e**

**Fig. 9 a**

**Fig 9 b**

**Fig 9 c**

**Fig. 9 d**

**Fig 9 e**

Fig. 10

<50>

<10>

<6,15,16,24>

<1>

Fig. 11 a,b

tissue

Cells

cm

ev

iv

$Z_{tissue}$     $R_{ev}$     $C_{cm}$     $R_{iv}$

Fig. 12

6,15,16,24

$Z_{generator}$

**Pulse generator**

$Z_{tissue}$

$U_{generator}$

6,15,16,24